(19) Europäisches Patentamt
European Patent Office
Office européen des brevets

(11) Numéro de publication : **0 113 293**
**B1**

(12) **FASCICULE DE BREVET EUROPÉEN**

(45) Date de publication du fascicule du brevet : **20.08.86**

(51) Int. Cl.[4] : **C 07 C121/75**, C 09 K 19/32

(21) Numéro de dépôt : **83402530.6**

(22) Date de dépôt : **23.12.83**

(54) **Famille de cristaux liquides présentant une phase smectique A, et son procédé de fabrication.**

(30) Priorité : **30.12.82 FR 8222074**

(43) Date de publication de la demande : **11.07.84 Bulletin 84/28**

(45) Mention de la délivrance du brevet : **20.08.86 Bulletin 86/34**

(84) Etats contractants désignés : **DE GB IT NL**

(56) Documents cités :
**DR.D.DEMUS et al.: "Flüssige Kristalle in Tabellen", 1975, VEB Deutscher Verlag für Grundstoffindustrie, Leipzig, DD**
**BULLETIN DE LA SOCIETE CHIMIQUE DE FRANCE, no. 9-10, 1975, pages 2066-2072, J. CANCEILL et al.: "Recherches sur les substances mésomorphes. VII. - Nouveaux matériaux smectiques dérivés du fluorène"**

(73) Titulaire : **THOMSON-CSF**
**173, Boulevard Haussmann**
**F-75379 Paris Cedex 08 (FR)**

(72) Inventeur : **Le Barny, Pierre**
**THOMSON-CSF SCPI 173, bld Haussmann**
**F-75379 Paris Cedex 08 (FR)**
Inventeur : **Dubois, Jean-Claude**
**THOMSON-CSF SCPI 173, bld Haussmann**
**F-75379 Paris Cedex 08 (FR)**
Inventeur : **Ravaux, Gilles**
**THOMSON-CSF SCPI 173, bld Haussmann**
**F-75379 Paris Cedex 08 (FR)**

(74) Mandataire : **Lepercque, Jean et al**
**THOMSON-CSF SCPI 19, avenue de Messine**
**F-75008 Paris (FR)**

**EP 0 113 293 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

L'invention concerne une famille de composés organiques du type ester de 2 hydroxy fluorène substitué, qui présente une phase mésomorphe de type smectique A. L'invention concerne également le procédé de fabrication des molécules de cette famille.

La famille de composés selon l'invention répond à la formule chimique générale :

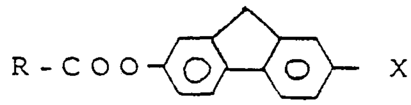

pour laquelle le radical R peut être :

$C_nH_{2n+1}-$ ,

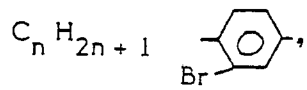

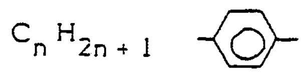 ,

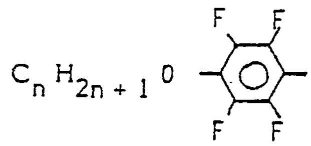 ou

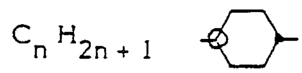

avec 1 ⩽ n ⩽ 15
et X = Br ou CN.

On peut désigner les composés de cette famille ainsi définie sous les dénominations de :

2 alcanoyloxy 7 bromo- ou cyano-fluorène,
2 [4' alkyl 3' bromobenzoyloxy] 7 bromo- ou cyano-fluorène,
2 [4' alkyl benzoyloxy] 7 bromo- ou cyano-fluorène,
2 [4' alkyloxy fluorobenzoyloxy] 7 bromo- ou cyano-fluorène,
2 [trans 4' alkylcyclohexane carbonyloxy] 7 bromo- ou cyano-fluorène.

La description qui va suivre portera sur le procédé général de synthèse des molécules selon l'invention ainsi que sur les propriétés mésomorphes des cristaux liquides correspondants.

Procédé général de synthèse

La famille organique selon l'invention présente un radical R qui est obtenu par des réactions chimiques assez différentes les unes des autres suivant les cas. Pour des raisons de clarté dans la description du procédé général de synthèse, on commencera par décrire l'étape donnant le produit final pour remonter ensuite aux produits de base et entrer dans les détails de fabrication.

1) synthèse des esters selon l'invention :

Réaction 1

La synthèse de ces esters se fait en une étape à partir du chlorure d'acide

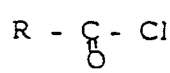

et d'un phénol (2 hydroxy 7 bromo- ou cyano-fluorène)

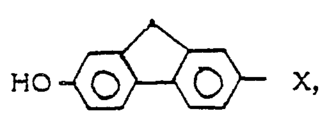

à température ambiante, dans la pyridine.

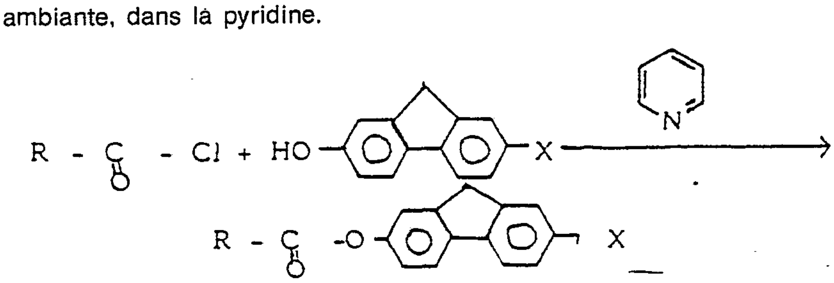

2) Obtention des chlorures d'acide

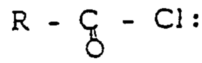

Réaction 2

Les chlorures d'alkanoyle et de 4 alkyl-benzoyle sont des produits accessibles pour l'homme de l'art. Les chlorures de 4 alkyl 3 bromobenzoyle, de 4 alkyloxy fluorobenzoyle et de 4 alkyl cyclohexane carbonyle sont préparés en une étape à partir des acides correspondants par action du chlorure de thionyle, selon la réaction classique :

$$R - COOH + SOCl_2 \xrightarrow{\text{reflux}} R - COCl + HCl\uparrow + SO_2\uparrow$$

3) Obtention des acides 4 alkyl 3 bromobenzoïques :

Réaction 3

Les acides 4 alkyl 3 bromobenzoïques sont préparés en une étape par un procédé connu (voir l'article de SPÄTH et PICKL publié dans la revue « BERICHTE der Deutschen Chemischen Gesellschaft », Berlin, 1929, p. 2259. On fait réagir du brome sur un acide alkylbenzoïque en présence de nitrate d'argent, d'acide nitrique et d'acide acétique à 25 °C.

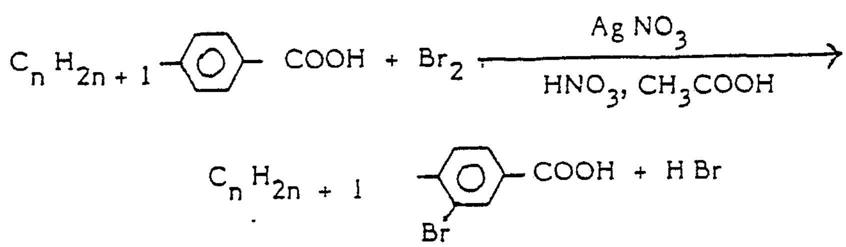


4) Obtention des acides 4 alkyloxy fluoro-benzoïques :

Réaction 4

Les acides 4 alkyloxy fluorobenzoïques sont obtenus en une étape à partir de l'acide fluorobenzoïque, selon un mode opératoire connu (voir BURDON, HOLLYHEAD, TATLOW, « Journal of Chemical Society », London, the Chemical Society, 1965, p. 6336, mais modifié afin d'en augmenter le rendement. La réaction consiste à faire réagir de l'alcoolate de sodium avec un acide pentafluorobenzoïque présent sous la forme d'un sel de sodium (le pentafluorobenzoate de sodium) dans un alcool qui sert de solvant puis en acidifiant le mélange réactionnel en fin de réaction.

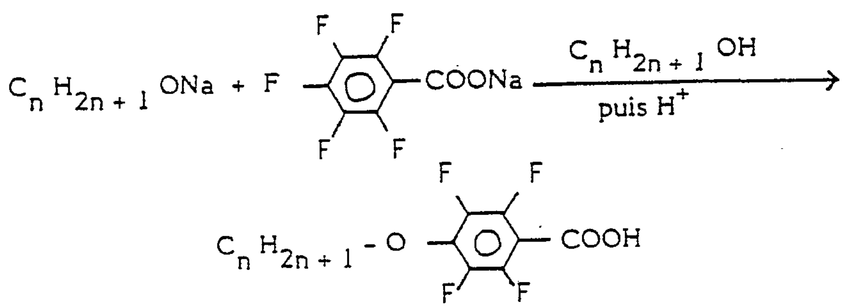


5) Obtention des acides trans alkyl cyclohexane carboxyliques :

Réaction 5

Ces acides sont préparés par hydrogénation catalytique des acides alkyl benzoïques selon la méthode de GRAY et McDONNELL (publiée en 1979 dans Molecular Crystal Liquid and Crystals n° 53, p. 147 à 166). La réaction s'effectue en présence de nickel de Raney W — 2, à 200 °C et sous une pression de 200 bars.

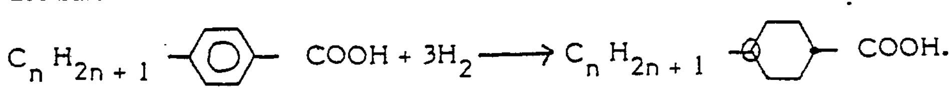

Le dérivé trans est séparé du dérivé cis par formation d'un composé d'inclusion avec la thiourée.

6) Obtention des 2 hydroxy 7 bromo- ou cyano-fluorène :

Le 2 hydroxy 7 bromo-fluorène est obtenu en six étapes à partir du fluorène selon le schéma réactionnel suivant :

— action de l'acide nitrique sur le fluorène en présence d'acide acétique à 80 °C :

Réaction 6

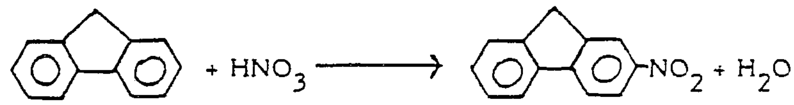

— action de l'acide chlorhydrique en présence de fer sur le composé organique obtenu par la réaction 6, dans de l'alcool éthylique à 90 % :

Réaction 7

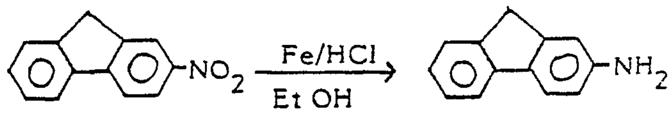


— action de l'acide nitreux sur le composé obtenu à la réaction 7, en présence de pyridine. Le produit final est obtenu par décomposition sous l'influence de la chaleur :

Réaction 8

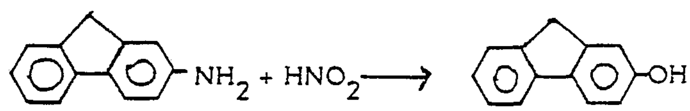

— action du chlorure d'acétyle sur le produit obtenu à la réaction 8, en présence de toluène et de pyridine :

Réaction 9

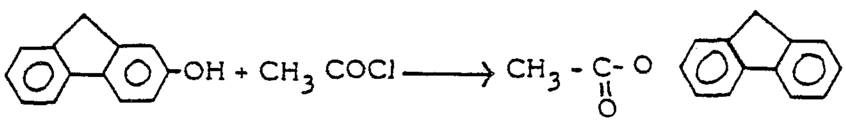

— action du brome sur le composé obtenu à la réaction 9 en présence d'acide acétique et d'anhydride acétique :

Réaction 10

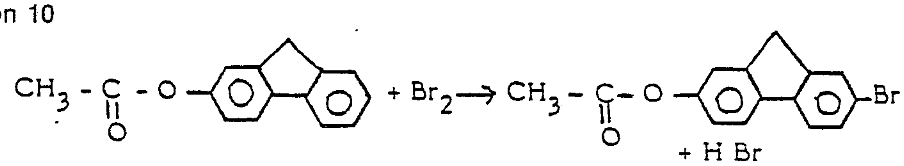

— action de l'hydroxyde de sodium puis de l'acide chlorhydrique sur le produit obtenu à la réaction 10 :

Réaction 11

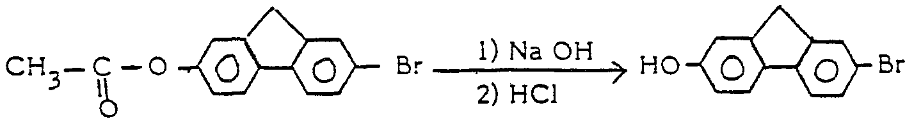


Le 2 hydroxy 7 cyano-fluorène est synthétisé à partir de 2 hydroxy 7 bromo-fluorène (obtenu à la réaction 11) par action du cyanure cuivreux dans le N méthyl pyrrolidone :

Réaction 12

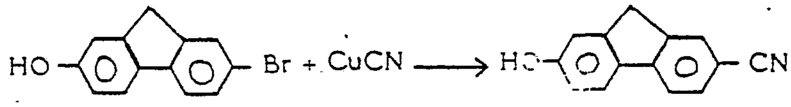

Les réactions 9, 11 et 12 sont classiques. Les réactions 6 et 7 ont été décrites respectivement par KUHN, « Organic Syntheses », New York, John Willey & Sons, Collective volume 2, p. 447 et par GRAY, HARTLEY et IBBOTSON (Journal of Chemical Society, London, The Chemical Society, 1955, p. 2686. La réaction 8 est inspirée du mode opératoire décrit dans « Methoden der organischen Chemie », (Houben-Weyl) Band VI/1c. Phenole Teil 1 ; KF Wedermeyer. p. 253 ; Georg Thieme Verlag, Stuttgart 1976.

Mode opératoire des réactions 1, 4, 8 et 10

1) Préparation générale des esters :

Réaction 1

Dans un erlenmeyer de 10 ml, on dissout 1,5 mM de 2 hydroxy 7 bromo- ou cyano-fluorène dans 3 ml de pyridine. La solution obtenue est versée dans un erlenmeyer contenant 1,5 mM de chlorure d'acide fraîchement préparé. Le milieu réactionnel est ensuite agité au moyen d'un barreau aimanté. On laisse la réaction se poursuivre à température ambiante pendant 48 heures. Le produit obtenu est alors versé sur environ 150 g d'un mélange eau-glace et on acidifie légèrement le milieu au moyen d'acide chlorhydrique titrant 25 %. Suit alors une phase d'extraction au chlorure de méthylène. On lave la phase organique à l'eau, puis on la sèche sur du sulfate de magnésium, on filtre et on évapore sous vide. Le produit brut est purifié par chromatographie liquide et soumis à l'éluant toluène 50-hexane 50, suivi par une recristallisation dans le cyclohexane.

2) Synthèse d'un acide 4 alkyloxy fluorobenzoïque :

Réaction 4

La réaction portera sur un exemple précis : la synthèse de l'acide 4 nonyloxyfluorobenzoïque, étant entendu que la synthèse des acides de la même famille peut être réalisée par la même méthode à partir des constituants de base correspondants.

Dans un erlenmeyer d'une contenance de 1 l pourvù d'un agitateur magnétique, on introduit 350 ml de nonanol 1 fraîchement distillé (afin d'introduire, dans la future molécule selon l'invention, une chaîne hydrocarbonée linéaire typique des cristaux liquides possédant une phase smectique), puis 4,6 g de sodium décapé. Le mélange est chauffé à 60 °C sous agitation jusqu'à disparition du sodium métallique.

Parallèlement, on dissout 21,2 g d'acide pentafluorobenzoïque dans 200 ml de nonanol 1 porté à 40 °C. La solution d'acide pentafluorobenzoïque est ajoutée à la solution de nonanoate de sodium. Le milieu réactionnel est agité pendant 48 heures à la température de 60 °C.

On laisse alors le milieu réactionnel revenir à la température ambiante, puis on le verse dans 800 ml d'eau permutée. Le milieu est ensuite acidifié à l'acide chlorhydrique titrant 25 % jusqu'à pH = 2.

La phase organique est extraite à l'éther, lavée à l'eau jusqu'à atteindre la neutralité, séchée sur le sulfate de magnésium, filtrée et évaporée sous vide.

Le nonanol en excès est éliminé par distillation sous vide. On recueille dans le bouilleur de distillation un liquide brun visqueux qui cristallise dans l'hexane à — 18 °C. Le précipité obtenu est filtré puis lavé à l'hexane froid et enfin recristallisé dans l'hexane. On obtient 4,93 g d'acide. Le rendement de la réaction est de 14,7 %. Le point de fusion du produit obtenu est F = 57 °C.

3) Synthèse du 2 hydroxy fluorène :

Réaction 8

Dans un réacteur d'une contenance de 1 l où l'on crée une agitation vigoureuse, on mélange 195 ml d'acide sulfurique à 95 ml d'eau. La solution d'acide sulfurique ainsi obtenue est ensuite refroidie à 0 °C au moyen d'un mélange acétone-carboglace. On ajoute par petites quantiés à cette solution, 19,5 g de nitrite de sodium. Le produit résultant se présente sous l'aspect d'un lait. On le chauffe au bain-marie, à une température de l'ordre de 40 à 50 °C, jusqu'à obtention d'une solution limpide jaune. Le milieu réactionnel est alors refroidi à 0 °C au moyen d'un mélange acétone-carboglace et on lui ajoute goutte à goutte en agitant, une solution de 23,5 g (0,13 mole) du produit obtenu à la réaction 7

(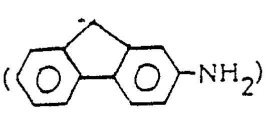)

dans 130 ml de pyridine. L'agitation est poursuivie à 0 °C pendant 1 heure. Le contenu du réacteur est ensuite transvasé dans un ballon de 4 l qui a été préalablement refroidi à — 18 °C et on y ajoute la quantié suffisante d'eau pour obtenir un volume total de 2,5 l. Puis on ajoute une solution aqueuse de 13 g d'urée et on poursuit l'agitation magnétique pendant 1 heure sous une température de 0 °C.

Parallèlement, on fait bouillir 5 l d'eau dans un réacteur de 10 l, puis on y ajoute rapidement le mélange réactionnel. L'ensemble est alors chauffé à reflux pendant 1 heure. Après retour à la température ambiante, un précipité apparaît que l'on filtre.

Le phénol brut que l'on obtient est dissous dans 2 l de soude (de concentration 10 %) portée à ébullition. Il se forme une substance de couleur marron, insoluble, que l'on filtre à chaud. Le filtrat chaud est acidifié à l'acide chlorhydrique (titrant 37 %) sous agitation. Le phénol précipite. Il est filtré, lavé à l'eau puis séché sous vide sur l'anhydride phosphorique $P_4O_{10}$. On obtient 15 g de phénol. Le rendement de la réaction est de 63,5 %. Le point de fusion du produit obtenu est F = 167 °C.

4) Synthèse du 2 acétoxy 7 bromofluorène :

Réaction 10

Dans un erlenmeyer de 500 ml où l'on réalise l'agitation magnétique et muni d'un réfrigérant à reflux et d'une ampoule à brome, on introduit 20 g de 2 acétyloxy-fluorène (89,3 mM) obtenu à l'issue de la réaction précédente, 110 ml d'acide acétique et 55 ml d'anhydride acétique. L'erlenmeyer est chauffé à 60 °C au moyen d'un bain d'huile afin de dissoudre l'ester, puis on ajoute 4,9 mg d'iode. On coupe le chauffage et on ajoute goutte à goutte 43 g de brome tout en agitant pendant 30 mn.

Après retour à la température ambiante, la solution se décolore. L'erlenmeyer est ensuite plongé dans un bain constitué d'eau et de glace. Un précipité apparaît alors. Il est filtré et la partie insoluble est lavée à l'hexane puis à l'eau. On obtient 18,17 mg de dérivé bromé blanc. De l'eau est ajoutée au filtrat et un précipité apparaît. Il est filtré, lavé à l'eau et séché. Le second précipité est purifié par recristallisation dans l'éther à — 18 °C, suivie d'une filtration sur la silice avec du benzène comme éluant. On obtient ainsi 1,56 g de 2 acétoxy 7 bromofluorène. Le rendement de la réaction est de 72,9 %. Le point de fusion du produit obtenu est F = 128 °C.

Propriétés des corps synthétisés

Les tableaux 1 et 2 ci-dessous rassemblent les résultats de l'étude calorimétrique effectuée sur quelques composés de la famille des esters de 2 bromo- ou cyano-fluorène selon l'invention. Suivant les radicaux R et X affectés aux molécules, on peut ainsi connaître à quelles températures s'effectuent les transitions entre les différentes phases : de la phase cristalline (K) à la phase liquide (L) en passant par la phase smectique $A(S_A)$ et éventuellement par la phase nématique (N). Les températures de transition sont exprimées en degrés Celsius. Les valeurs entre parenthèses sont des températures correspondant à des phases monotropiques. Les valeurs entre crochets sont les enthalpies de transition exprimées en kcal/mole.

La nature des mésophases a été déterminée par l'étude de l'isomorphisme au microscope optique des composés étudiés avec des substances connues.

Tous les esters figurés dans les tableaux présentent une mésophase smectique A, parfois monotropique, et dans certains cas une phase nématique.

La lecture du tableau 1 montre que l'ester n° 1 (correspondant à $R = C_7H_{15}-$ et X = Br) présente une phase smectique A monotropique. Lorsque la température de ce cristal s'élève, il passe directement de la phase cristalline à la phase liquide, la transition de phase s'effectuant à 101 °C. En se refroidissant, le cristal revient à la phase cristalline en présentant une phase smectique pour une température de 98,5 °C. L'ester n° 4 ($R = C_9H_{19}-$, X = CN) présente également une phase smectique monotropique.

Certains esters tels que les numéros 10, 11, 12 et 13 présentent en plus de la phase smectique, une phase nématique (tableau 2).

L'étude calorimétrique a démontré que les 2 alcanoyloxy 7 bromofluorènes

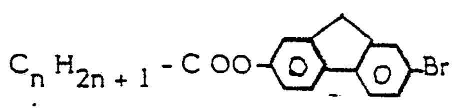

ne sont pas mésomorphes pour N < 7, et que les 2 alcanoyloxy 7 cyanofluorènes

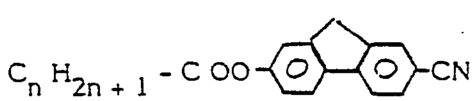

ne sont pas mésomorphes pour n < 9. Par contre, les autres esters selon l'invention présentent bien une phase smectique pour des valeurs de n comprises entre 1 et 15.

Il entre dans le cadre de l'invention d'utiliser les composés organiques ainsi définis comme cristaux liquides smectiques, seuls ou en mélange entre eux ou avec d'autres produits, ce mélange présentant également une phase smectique. A ce titre, ils peuvent être utilisés dans des dispositifs de visualisation.

Tableau 1

| N° | R | X | K | | $S_A$ | | L |
|---|---|---|---|---|---|---|---|
| 1 | $C_7H_{15}$– | Br | x<br> | | <br>x | 101 [5,49]<br>(98,5) [1,14] | x<br>x |
| 2 | $C_8H_{17}$– | Br | x | 85,3 [4,13] | x | 92,4 [1,14] | x |
| 3 | $C_9H_{19}$– | Br | x | 85 [4,80] | x | 95 [1,3] | x |
| 4 | $C_9H_{19}$– | CN | x<br> | | <br>x | 71,9 [8,81]<br>(69) [0,93] | x<br>x |
| 5 | $C_{11}H_{23}$– | CN | x | 81 [8,16] | x | 84,2 [0,80] | x |
| 6 | $C_{13}H_{27}$– | CN | x | 76,4 [8,07] | x | 91,5 [0,92] | x |
| 7 | $C_{14}H_{29}$– | CN | x | 80,4 [9,17] | x | 92,9 [1,12] | x |
| 8 | $C_{10}H_{21}$ 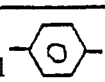 | CN | x | 125,5 [6,57] | x | 192 [0,60] | x |
| 9 | $C_7H_{15}$ 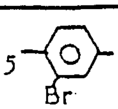 | Br | x | 107,8 [7,10] | x | 139,3 [0,78] | x |

Tableau 2

| N° | R | X | K | | $S_A$ | | N | | L |
|---|---|---|---|---|---|---|---|---|---|
| 10 | $C_9H_{19}$ 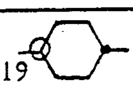 | CN | x | 96 [4,68] | X | 209 [0,17] | X | 210 [0,14] | X |
| 11 | $C_7H_{15}$ 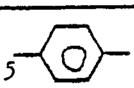 | CN | x | 140,5 [6,32] | X | 153 | X | 204,8 [0,16] | X |
| 12 | $C_8H_{17}$ 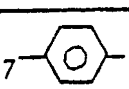 | CN | x | 134,8 [6,51] | X | 182,5 [0,03] | X | 198,5 [0,12] | X |
| 13 | $C_9H_{19}$ 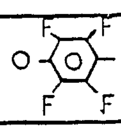 | CN | x | 109 [11,54] | X | 140,5 [0,05] | X | 152,5 [0,11] | X |

**Revendications**

1. Composé organique du type ester de fluorène substitué, présentant au moins une phase mésomorphe de type smectique A, caractérisé en ce qu'il répond à la formule chimique générale :

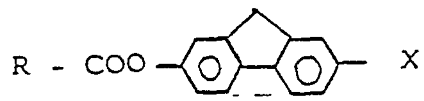

pour laquelle R est soit :

— $C_nH_{2n+1}$- avec $7 \leq n \leq 15$ et X = Br

— $C_nH_{2n+1}$- avec $9 \leq n \leq 15$ et X = CN

— $C_nH_{2n+1}$ 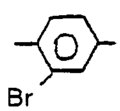 avec $1 \leq n \leq 15$

et X = Br ou CN

— $C_nH_{2n+1}$ 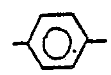 avec $1 \leq n \leq 15$

et X = Br ou CN

— $C_nH_{2n+1}$ 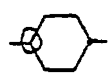 avec $1 \leq n \leq 15$

X = Br ou CN

— $C_nH_{2n+1}$ O 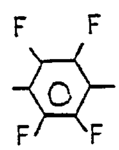 avec $1 \leq n \leq 15$

et X = Br ou CN.

2. Composé organique selon la revendication 1, caractérisé en ce que :

R = $C_8H_{17}$- et X = Br.

3. Composé organique selon la revendication 1, caractérisé en ce que :

R = $C_9H_{19}$- et X = Br.

4. Composé organique selon la revendication 1, caractérisé en ce que :

R = $C_{11}H_{23}$- et X = CN.

5. Composé organique selon la revendication 1, caractérisé en ce que :

R = $C_{13}H_{27}$- et X = CN.

6. Composé organique selon la revendication 1, caractérisé en ce que :

R = $C_{14}H_{29}$- et X = CN.

7. Composé organique selon la revendication 1, caractérisé en ce que :

R = $C_7H_{15}$ 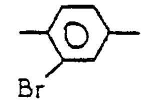 et X = Br.

8. Composé organique selon la revendication 1, caractérisé en ce que :

R = $C_{10}H_{21}$ 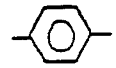 et X = CN.

9. Composé organique selon la revendication 1, caractérisé en ce que :

R = $C_9H_{19}$ 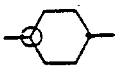 et X = CN,

ledit composé présentant également une phase nématique.

10. Composé organique selon la revendication 1, caractérisé en ce que :

R = $C_7H_{15}$ 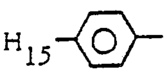 et X = CN,

ledit composé présentant également une phase nématique.

11. Composé organique selon la revendication 1, caractérisé en ce que :

R = $C_8H_{17}$ 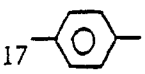 et X = CN,

ledit composé présentant également une phase nématique.

12. Composé organique selon la revendication 1, caractérisé en ce que :

R = $C_9H_{19}O$ 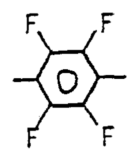 et X = CN,

ledit composé présentant également une phase nématique.

13. Composition d'un mélange de cristaux liquides, ledit mélange présentant au moins une phase smectique, caractérisée en ce qu'elle contient un composé organique selon l'une quelconque des revendications 1 à 12.

14. Procédé de fabrication d'un composé organique selon la revendication 1, caractérisé en ce que ledit composé est un produit de la réaction du chlorure d'acide

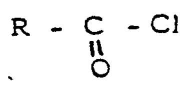

et du phénol

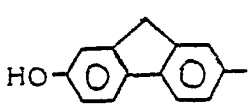 X,

ladite réaction s'effectuant à température ambiante, dans la pyridine.

15. Procédé de fabrication selon la revendication 14, caractérisé en ce que la synthèse dudit phénol a été réalisée au cours des étapes séquentielles suivantes :

a) synthèse du 2 hydroxy fluorène par addition d'une solution de pyridine contenant du 2 aminofluorène dans de l'acide nitreux refroidi à 0 °C ;

b) acétylation du produit obtenu à l'étape précédente en présence de pyridine ;

c) bromation du produit obtenu à l'étape (b) dans un mélange acide acétique-anhydride acétique ;

d) saponification du produit obtenu à l'étape (c).

16. Procédé de fabrication selon la revendication 15, caractérisé en ce que la synthèse dudit phénol comprend en outre une étape de cyanuration du produit obtenu à l'étape (d) dans le N méthyl pyrrolidone.

17. Dispositif de visualisation à cristal liquide smectique, caractérisé en ce que ledit cristal liquide comprend au moins un composé organique selon l'une quelconque des revendications 1 à 12.

**Claims**

1. Organic compound of the substituted fluorene ester type having at least one mesomorphous phase of the type smectic A characterized in that it satisfies the general chemical formula :

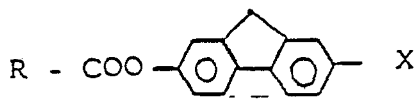

wherein R is :

— $C_nH_{2n+1}$– with 7 ⩽ n ⩽ 15 and X = Br

— $C_nH_{2n+1}$– with 9 ⩽ n ⩽ 15 and X = CN

— $C_nH_{2n+1}$ 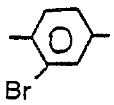 with 1 ⩽ n ⩽ 15

and X = Br or CN

— $C_nH_{2n+1}$ 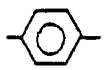 with 1 ⩽ n ⩽ 15

and X = Br or CN

— $C_nH_{2n+1}$ 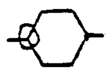 with 1 ⩽ n ⩽ 15

X = Br or CN

— $C_nH_{2n+1}$ O 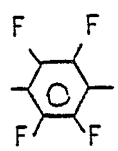 with 1 ⩽ n ⩽ 15

and X = Br or CN.

2. The organic compound of claim 1 wherein

R = $C_8H_{17}$– and X = Br.

3. The organic compound of claim 1 wherein

R = $C_9H_{19}$– and X = Br.

4. The organic compound of claim 1 wherein

R = $C_{11}H_{23}$– and X = CN.

5. The organic compound of claim 1 wherein

R = $C_{13}H_{27}$– and X = CN.

6. The organic compound of claim 1 wherein

R = $C_{14}H_{29}$– and X = CN.

7. The organic compound of claim 1 wherein

R = $C_7H_{15}$ 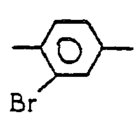 and X = Br.

8. The organic compound of claim 1 wherein

R = $C_{10}H_{21}$ 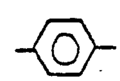 and X = CN.

9. The organic compound of claim 1 wherein

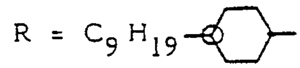 and X = CN,

said compound also having a nematic phase.

10. The organic compound of claim 1 wherein

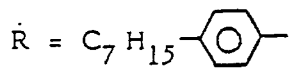 and X = CN,

said compound also having a nematic phase.

11. The organic compound of claim 1 wherein

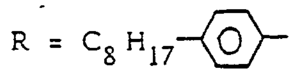 and X = CN,

said compound also having a nematic phase.

12. The organic compound of claim 1 wherein

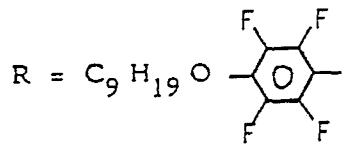 and X = CN,

said compound also having a nematic phase.

13. Composition of a mixture of liquid crystals with said mixture having at least a smectic phase characterized in that it contains an organic compound according to any one of claims 1 to 12.

14. Process for the manufacture of an organic compound according to claim 1 characterized in that said compound being a product of the reaction of the acid chloride.

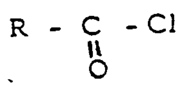

and of the phenol

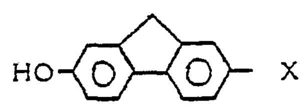

with said reaction being effected at ambient temperature in pyridine.

15. The process of claim 14 characterized in that the synthesis of said phenol has been realized in the course of the following sequential steps :

a) synthesis of 2-hydroxy fluorene by addition of a solution of pyridine containing 2-amino fluorene in nitric acid refrigerated to 0 °C ;

b) acetylation of the product obtained from the preceding step in the presence of pyridine ;

c) bromination of the product obtained in step (b) in an acetic acid-acetic anhydride mixture ;

d) saponification of the product obtained in step (c).

16. The process of claim 15 characterized in that the synthesis of said phenol additionally comprises a step of cyanidation the product obtained in step (d) in N-methyl pyrrolidone.

17. Smectic liquid crystal display characterized in that said liquid crystal comprises at least one organic compound according to any one of claims 1 to 12.

**Patentansprüche**

1. Organische Verbindung vom Typ substituierter Fluorenester, die mindestens eine mesomorphe smektische Phase vom Typ A aufweist, dadurch gekennzeichnet, daß sie der allgemeinen chemischen Formel :

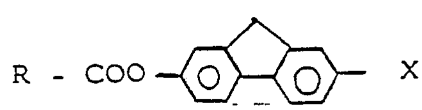

entspricht, worin R die folgenden Bedeutungen besitzt :

— $C_nH_{2n+1}$- mit $7 \leqslant n \leqslant 15$ und X = Br

— $C_nH_{2n+1}$- mit $9 \leqslant n \leqslant 15$ und X = CN

— $C_nH_{2n+1}$ 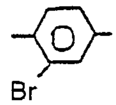 mit $1 \leq n \leq 15$

und X = Br oder CN

— $C_nH_{2n+1}$ 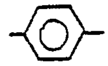 mit $1 \leq n \leq 15$

und X = Br oder CN

— $C_nH_{2n+1}$ 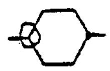 mit $1 \leq n \leq 15$

X = Br oder CN

— $C_nH_{2n+1}$ O 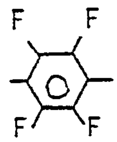 mit $1 \leq n \leq 15$

und X = Br oder CN.

2. Organische Verbindung nach Anspruch 1, dadurch gekennzeichnet, daß

$$R = C_8H_{17}- \text{ und } X = Br$$

bedeuten.

3. Organische Verbindung nach Anspruch 1, dadurch gekennzeichnet, daß

$$R = C_9H_{19}- \text{ und } X = Br$$

bedeuten.

4. Organische Verbindung nach Anspruch 1, dadurch gekennzeichnet, daß

$$R = C_{11}H_{23}- \text{ und } X = CN$$

bedeuten.

5. Organische Verbindung nach Anspruch 1, dadurch gekennzeichnet, daß

$$R = C_{13}H_{27}- \text{ und } X = CN$$

bedeuten.

6. Organische Verbindung nach Anspruch 1, dadurch gekennzeichnet, daß

$$R = C_{14}H_{29}- \text{ und } X = CN$$

bedeuten.

7. Organische Verbindung nach Anspruch 1, dadurch gekennzeichnet, daß

R = $C_7H_{15}$ 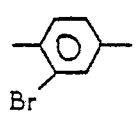 und X = Br

bedeuten.

8. Organische Verbindung nach Anspruch 1, dadurch gekennzeichnet, daß

R = $C_{10}H_{21}$ 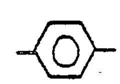 und X = CN

bedeuten.

9. Organische Verbindung nach Anspruch 1, dadurch gekennzeichnet, daß

R = $C_9H_{19}$ 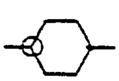 und X = CN

bedeuten und daß die genannte Verbindung auch eine nematische Phase aufweist.

10. Organische Verbindung nach Anspruch 1, dadurch gekennzeichnet, daß

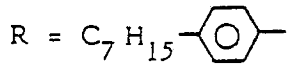 und X = CN

bedeuten und daß die genannte Verbindung auch eine nematische Phase aufweist.

11. Organische Verbindung nach Anspruch 1, dadurch gekennzeichnet, daß

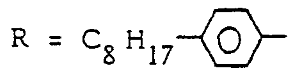 und X = CN

bedeuten und die genannte Verbindung auch eine nematische Phase aufweist.

12. Organische Verbindung nach Anspruch 1, dadurch gekennzeichnet, daß

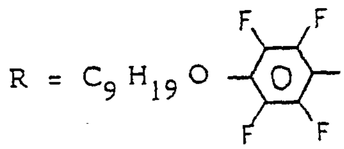 und X = CN

bedeuten und die genannte Verbindung auch eine nematische Phase aufweist.

13. Aus einem Gemisch von Flüssigkristallen bestehende Zusammensetzung, wobei das Gemisch mindestens eine smektische Phase aufweist, dadurch gekennzeichnet, daß sie eine organische Verbindung gemäß einem der Ansprüche 1 bis 12 enthält.

14. Verfahren zur Herstellung einer organischen Verbindung gemäß Anspruch 1, dadurch gekennzeichnet, daß die Verbindung ein Produkt der Reaktion des Säurechlorids

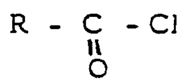

mit dem Phenol

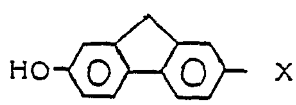

ist und daß die Reaktion bei Raumtemperatur in Pyridin durchgeführt wird.

15. Verfahren nach Anspruch 14, dadurch gekennzeichnet, daß die Synthese des Phenols im Verlaufe der folgenden aufeinanderfolgenden Stufen durchgeführt worden ist :

a) Synthese von 2-Hydroxyfluoren durch Zugabe einer 2-Aminofluoren enthaltenden Pyridinlösung zu auf 0 °C gekühlter Salpetersäure ;

b) Acetylierung des in der vorgenannten Stufe erhaltenen Produkts in Gegenwart von Pyridin ;

c) Bromierung des in Stufe (b) erhaltenen Produkts in einem Gemisch aus Essigsäure und Acetanhydrid ;

d) Verseifung des in Stufe (c) erhaltenen Produkts.

16. Verfahren nach Anspruch 15, dadurch gekennzeichnet, daß die Synthese des Phenols zusätzlich eine Stufe zur Einführung des Cyanidrestes in das in Stufe (d) erhaltene Produkt in N-Methylpyrrolidon umfaßt.

17. Smektische Flüssigkristallanzeige, dadurch gekennzeichnet, daß der Flüssigkristall mindestens eine organische Verbindung gemäß einem der Ansprüche 1 bis 12 umfaßt.